(19) Europäisches Patentamt
European Patent Office
Office européen des brevets

(11) **EP 2 065 471 B1**

(12) **EUROPEAN PATENT SPECIFICATION**

(45) Date of publication and mention
of the grant of the patent:
**16.08.2017 Bulletin 2017/33**

(51) Int Cl.:
*C12P 13/08* (2006.01)  *C12P 13/10* (2006.01)
*C12P 13/24* (2006.01)  *C12P 13/04* (2006.01)
*C07C 229/26* (2006.01)  *C07C 229/28* (2006.01)
*C07D 233/64* (2006.01)

(21) Application number: **09001098.4**

(22) Date of filing: **23.08.2001**

(54) **Method for producing basic amino acid**

Verfahren zur Herstellung einer basischen Aminosäure

Procédé de production d'acide aminé basique

(84) Designated Contracting States:
**DE DK FR GB NL**

(30) Priority: **24.08.2000 JP 2000253692**

(43) Date of publication of application:
**03.06.2009 Bulletin 2009/23**

(62) Document number(s) of the earlier application(s) in
accordance with Art. 76 EPC:
**01120248.8 / 1 182 261**

(73) Proprietor: **Ajinomoto Co., Inc.**
**Tokyo 104-8315 (JP)**

(72) Inventors:
• **Kobayashi, Masaki**
**Kawasaki-shi**
**Kanagawa (JP)**
• **Itoyama, Tsuyoshi**
**Kawasaki-shi**
**Kanagawa (JP)**
• **Mitani, Yukio**
**Tokyo (JP)**
• **Usui, Naoki**
**Kawasaki-shi**
**Kanagawa (JP)**

(74) Representative: **Hoffmann Eitle**
**Patent- und Rechtsanwälte PartmbB**
**Arabellastraße 30**
**81925 München (DE)**

(56) References cited:
EP-A- 0 532 867    EP-A- 0 564 672
EP-A2- 0 809 940    DD-A- 290 213
US-A- 4 275 157    US-A- 5 431 933
US-A- 5 650 304    US-A- 5 770 409
US-A- 6 025 169

EP 2 065 471 B1

**Description**

Background of the Invention

Field of the Invention

The present invention relates to a method for producing a basic amino acid by fermentation and a fermentation product of basic amino acid. Basic amino acids are useful, for example, L-lysine is useful as an additive for animal feed and L-arginine and L-histidine are useful for pharmaceutical preparations such as infusions.

Description of the Related Art

In the methods for producing basic amino acids by fermentation, microorganisms having an ability to produce a basic amino acid are cultured to produce and accumulate the basic amino acid in culture broth, and the basic amino acid is collected from the culture broth. In such methods, the culture is performed as batch culture, feeding culture or continuous culture.

In such production of basic amino acids by fermentation, sulfate ions or chloride ions have been conventionally added to a medium as counter anions in order to maintain electrical neutrality. As for a source of sulfate ions, they are mainly supplied as ammonium sulfate (Japanese Patent Laid-open Publication (Kokai) Nos. 5-30985 and 5-244969).

In most cases, the collection of basic amino acids from culture broth is performed by an ion exchange method, when purification is required. For example, in the case of L-lysine, after fermentation broth is made weekly acidic, L-lysine is adsorbed on an ion exchange resin and then desorbed from the resin with ammonium ions. The desorbed L-lysine is used as it is as lysine base, or crystallized as L-lysine hydrochloride with hydrochloric acid.

On the other hand, when they are not purified, the fermentation broth is concentrated as it is, or after it is made weekly acidic with hydrochloric acid or sulfuric acid, it is subjected to spray granulation. In this case, since the content of the basic amino acid in the obtained fermentation product is restricted by residual components contained in the medium, the counter anions added to the medium cannot be ignored. Therefore, reduction of the amount of the counter anions to be used has an important significance in view of not only production cost but also quality of product.

By the way, microorganisms discharge a lot of carbon dioxide gas during fermentation through physiological metabolism such as respiration. Japanese Patent Laid-open Publication No. 11-243985 discloses a method of collecting carbon dioxide gas in L-glutamic acid fermentation characterized by allowing sodium hydrogencarbonate and/or sodium carbonate to act on L-glutamic acid separated and obtained from L-glutamic acid fermentation broth in an aqueous medium to produce monosodium L-glutamate and, at the same time, collecting carbon dioxide gas produced as a secondary product. However, the carbon dioxide gas is not effectively used during the fermentation.

Summary of the Invention

An object of the present invention is to provide a technique for reducing an amount of counter anion source to be added to a medium during the production of basic amino acids by fermentation.

The inventors of the present invention found that the amount of the counter anion source could be reduced and carbon dioxide gas produced during fermentation can be effectively used by utilizing the carbon dioxide gas in place of counter anions such as sulfate ions, and thus accomplished the present invention.

That is, the present invention provides the following.

(1) A fermentation broth containing a basic amino acid obtainable by culturing a microorganism having an ability to produce the basic amino acid in a liquid medium under an aerobic condition, or a concentrate thereof, wherein the fermentation broth and the concentrate thereof contain hydrogencarbonate ions and/or carbonate ions as counter-ions of cations mainly consisting of the basic amino acid in a normality ratio of 5% to 80%, which ratio is calculated according to the following equation:

```
normality ratio = [normality of hydrogencarbonate ions
and/or carbonate ions] / [normality of cations mainly
consisting of basic amino acid] x 100.
```

(2) The fermentation broth containing a basic amino acid or the concentrate thereof according to (1), wherein the basic amino acid consists of one or more kinds of amino acids selected from L-lysine, L-arginine and L-histidine.

(3) The fermentation broth containing a basic amino acid or the concentrate thereof according to any one of (1) and (2), wherein the microorganism is coryneform bacterium or *Escherichia coli.*

(4) The fermentation broth containing a basic amino acid or the concentrate thereof according to (3), wherein the coryneform bacterium is *Corynebacterium glutamicum.*

(5) A method for producing the fermentation broth containing a basic amino acid according to any one of (1) to (4) by fermentation, comprising
the step of culturing a microorganism having an ability to produce the basic amino acid in a liquid medium under an aerobic condition to produce and accumulate the basic amino acid in the medium, wherein:

pH of the medium is controlled to be 6.5-9.0 during the culture, and 7.2-9.0 at the end of the culture, and

a culture period where 2 g/L or more of hydrogencarbonate ions and/or carbonate ions exist in the medium is secured during the culture by controlling pressure in a fermentation tank to be a positive pressure during the fermentation, or supplying carbon dioxide gas or a mixed gas containing carbon dioxide gas to the medium, wherein the pressure in the fermentation tank is 0.03-0.2 MPa,

so that the hydrogencarbonate ions and/or carbonate ions should serve as counter ions of cations mainly consisting of the basic amino acid.

(6) The method according to (5), wherein the basic amino acid consists of one or more kinds of amino acids selected from L-lysine, L-arginine and L-histidine.

(7) The method according to any one of (5) and (6), wherein the microorganism is coryneform bacterium or *Escherichia coli.*

(8) The method according to (7), wherein the coryneform bacterium is *Corynebacterium glutamicum.*

Additionally the following is disclosed:

(1) A method for producing a basic amino acid, or fermentation broth or fermentation product containing the basic amino acid by fermentation comprising the step of culturing a microorganism having an ability to produce the basic amino acid in a liquid medium under an aerobic condition to produce and accumulate the basic amino acid in the medium, wherein:

pH of the medium is controlled to be 6.5-9.0 during the culture, and 7.2-9.0 at the end of the culture, and
a culture period where 2 g/L or more of hydrogencarbonate ions and/or carbonate ions exist in the medium is secured during the culture by controlling pressure in a fermentation tank to be a positive pressure during the fermentation, or supplying carbon dioxide gas or a mixed gas containing carbon dioxide gas to the medium, so that the hydrogencarbonate ions and/or carbonate ions should serve as counter ions of cations mainly consisting of the basic amino acid.

(2) The method for producing a basic amino acid, or fermentation broth or fermentation product containing the basic amino acid according to (1), wherein the pressure in the fermentation tank is 0.03-0.2 MPa.
(3) The method for producing a basic amino acid, or fermentation broth or fermentation product containing the basic amino acid according to (1) or (2), wherein the basic amino acid consists of one or more kinds of amino acids selected from L-lysine, L-arginine and L-histidine.
(4) A fermentation broth containing a basic amino acid obtained by culturing a microorganism having an ability to produce the basic amino acid in a liquid medium under an aerobic condition or a fermentation product produced from the fermentation broth, which contains hydrogencarbonate ions and/or carbonate ions as counter ions of cations mainly consisting of the basic amino acid in a normality ratio of 5-80%, which ratio is calculated according to the following equation:

```
Normality ratio =

[Normality of hydrogencarbonate ions and/or

carbonate ions]/[Normality of cations mainly consisting of basic amino acid] x 100
```

(5) The fermentation broth containing a basic amino acid or fermentation product according to (4), wherein the basic amino acid consists of one or more kinds of amino acids selected from L-lysine, L-arginine and L-histidine.

(6) A fermentation broth or fermentation product containing a basic amino acid, which is obtained by allowing the fermentation broth containing a basic amino acid or the fermentation product according to (4) to discharge carbon dioxide gas.

According to the present invention, the amounts of industrial materials such as ammonium sulfate can be reduced, and hence basic amino acids can be produced at a low cost. Further, although the obtained fermentation broth contains carbonate ions and/or hydrogencarbonate ions, they can be easily discharged into atmosphere by heating. Therefore, a fermentation broth or fermentation product having a high amino acid concentration in solid content can be obtained.

Brief Explanation of the Drawings

Fig. 1 shows time course of normality ratios of sulfate ions as well as carbonate ions and hydrogencarbonate ions with respect to cations mainly consisting of lysine in culture broth and pressure in fermentation tank obtained in Example 1 and Comparative Example 1.

Fig. 2 shows time course of normality ratios of sulfate ions as well as carbonate ions and hydrogencarbonate ions with respect to cations mainly consisting of lysine in culture broth and pressure in fermentation tank obtained in Example 2 and Comparative Example 2.

Detailed Description of the Invention

The present invention will be explained in detail hereafter.

The method of the present invention is characterized by utilizing either one or both of carbonate ions and hydrogencarbonate ions as major counter ions of basic amino acid in a method for producing a fermentation broth, or a concentrate thereof, comprising culturing a microorganism having an ability to produce the basic amino acid in a liquid medium under an aerobic condition to produce and accumulate the basic amino acid in the medium.

The basic amino acid produced from the fermentation broth, or concentrate thereof, of the present invention may consist of, for example, one or more kinds of amino acids selected from L-lysine, L-arginine and L-histidine.

The microorganism having an ability to produce a basic amino acid used in the method of the present invention is not particularly limited, and any microorganisms can be used so long as they can produce a basic amino acid by fermentation. Examples of such microorganisms include, for example, coryneform bacteria, bacteria belonging to the genus *Escherichia, Serratia, Bacillus* and so forth. While coryneform bacteria and *Escherichia* bacteria will be explained below, the microorganism used for the method of the present invention is not limited to these bacteria.

Coryneform bacteria include those having hitherto been classified into the genus *Brevibacterium,* but united into the genus *Corynebacterium* at present (Int. J. Syst. Bacteriol., 41, 255 (1981)), and include bacteria belonging to the genus *Brevibacterium* closely relative to the genus *Corynebacterium.* Examples of such coryneform bacteria are mentioned below.

*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium (Corynebacterium glutamicum)*
*Corynebacterium melassecola*
*Corynebacterium thermoaminogenes*

*Corynebacterium herculis*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Brevibacterium album*
*Brevibacterium cerinum*
*Microbacterium ammoniaphilum*

As an example of the bacteria belonging to the genus *Escherichia, Escherichia coli* can be mentioned.

Examples of the coryneform bacteria having L-lysine producing ability include mutant strains resistant to S-(2-aminoethyl)-cysteine (abbreviated as "AEC" hereinafter), mutant strains requiring amino acids such as L-homoserine for their growth (Japanese Patent Publication (Kokoku) Nos. 48-28078 and 56-6499), mutant strains resistant to AEC and further requiring amino acids such as L-leucine, L-homoserine, L-proline, L-serine, L-arginine, L-alanine and L-valine (U.S. Patent Nos. 3,708,395 and 3,825,472), L-lysine producing mutant strains resistant to DL-$\alpha$-amino-$\epsilon$-caprolactam, $\alpha$-amino-lauryllactam, aspartic acid analogue, sulfa drug, quinoid and N-lauroylleucine, L-lysine producing mutant strains resistant to oxaloacetate decarboxylase or a respiratory system enzyme inhibitor (Japanese Patent Laid-open Nos. 50-53588, 50-31093, 52-102498, 53-9394, 53-86089, 55-9783, 55-9759, 56-32995, 56-39778, Japanese Patent Publication Nos. 53-43591 and 53-1833), L-lysine producing mutant strains requiring inositol or acetate (Japanese Patent Laid-open Nos. 55-9784 and 56-8692), L-lysine producing mutant strains that are sensitive to fluoropyruvic acid or a temperature of 34°C or higher (Japanese Patent Laid-open Nos. 55-9783 and 53-86090), L-lysine producing mutant strains of *Brevibacterium* or *Corynebacterium* bacteria resistant to ethylene glycol (U.S. Patent Application No. 333,455) and so forth.

Specific examples are *Brevibacterium lactofermentum* ATCC31269, *Brevibacterium flavum* ATCC21475 and *Corynebacterium acetoglutamicum* ATCC21491.

As L-lysine producing bacteria belonging to the genus *Escherichia,* mutant strains resistant to an L-lysine analogue can be exemplified. This L-lysine analogue is a substance that inhibits growth of bacteria belonging to the genus *Escherichia* bacteria, but this inhibition is fully or partially desensitized if L-lysine exists in the medium. For example, oxalysine, lysine hydroxamate, (S)-2-aminoethyl-L-cysteine (AEC), $\gamma$-methyllysine, $\alpha$-chlorocaprolactam and so forth can be mentioned. A mutant strain resistant to these lysine analogues can be obtained from microorganisms of the genus *Escherichia* by a usual artificial mutation technique. Specific examples of bacterial strains used for L-lysine production include *Escherichia coli* AJ11442 (FERM BP-1543, NRRL B-12185; see Japanese Patent Laid-open Publication No. 56-18596 and U.S. Patent No. 4,346,170) and *Escherichia coli* VL611. The AJ11442 strain is deposited at the National Institute of Bioscience and Human-Technology, Agency of Industrial Science and Technology, Ministry of International Trade and Industry (currently, the independent administrative corporation, National Institute of Advanced Industrial Science and Technology, International Patent Organism Depositary)(Chuo Dai-6, 1-1 Higashi 1-Chome, Tsukuba-shi, Ibaraki-ken, Japan, postal code: 305-5466) on May 1, 1981, and received an accession number of FERM P-5084. Then, it was transferred from the above original deposit to an international deposit under the provisions of the Budapest Treaty on October 29, 1987 and received an accession number of FERM BP-1543. In the aforementioned microorganisms, the feedback inhibition of aspartokinase by L-lysine is desensitized.

Specific examples of *Escherichia coli* strains having L-lysine producing ability include *Escherichia coli* W3110(tyrA)/pCABD2 (International Patent Publication WO95/16042) and so forth. The *Escherichia coli* W3110(tyrA)/pCABD2 is a strain obtained by introducing a plasmid pCABD2 containing L-lysine biosynthesis system enzyme genes into W3110(tyrA), which is a *tyrA* deficient strain of *Escherichia coli.* The strain W3110(tyrA), which is designated as AJ12604, was deposited at the National Institute of Bioscience and Human-Technology, Agency of Industrial Science and Technology, Ministry of International Trade and Industry (currently, the independent administrative corporation, National Institute of Advanced Industrial Science and Technology, International Patent Organism Depositary) (Chuo Dai-6, 1-1 Higashi 1-Chome, Tsukuba-shi, Ibaraki-ken, Japan, postal code: 305-5466) on January 28, 1991 under an accession number of FERM P-11975, and then transferred to an international deposit under the provisions of the Budapest Treaty on October 29, 1987 under an accession number of FERM BP-3579).

The plasmid pCABD2 contains a gene coding for a mutant type dihydrodipicolinate synthase of which 118th histidine residue is replaced with a tyrosine residue and feedback inhibition by L-lysine is desensitized, gene coding for a mutant type aspartokinase III of which 352nd threonine residue is replaced with an isoleucine residue and feedback inhibition by L-lysine is desensitized, and genes coding for dihydrodipicolinate reductase and diaminopimelate dehydrogenase.

Further, the *E. coli* W3110(tyrA) strain can be obtained as described below. That is, many strains obtained by introducing a plasmid into the W3110(tyrA) strain are disclosed in European Patent Laid-open Publication No. 488424/1992. For example, a strain obtained by introducing a plasmid pHATerm is designated as *E. coli* W3110(tyrA)/pHATerm strain, and deposited at the National Institute of Bioscience and Human-Technology, Agency of Industrial Science and Technology (currently, the independent administrative corporation, National Institute of Advanced Industrial Science and Technology, International Patent Organism Depositary) under an accession number of FERM BP-3653. The W3110(tyrA) strain can be obtained by, for example, eliminating the plasmid pHATerm from this *E. coli* W3110(tyrA)/pHATerm strain. Elimination of the plasmid can be performed in a conventional manner.

Examples of L-lysine producing bacteria belonging to the genus *Serratia* include *Serratia* bacteria transformed by introduction of a DNA coding for dihydrodipicolinate synthase having a mutation that desensitizes feedback inhibition by L-lysine into their cells, and *Serratia* bacteria containing aspartokinase of which feedback inhibition by L-lysine is desensitized (WO96/41871).

Examples of coryneform bacteria having an ability to producing L-arginine include wild-type strains of coryneform bacteria; coryneform bacteria resistant to certain agents including sulfa drugs, 2-thiazolealanine, $\alpha$-amino-$\beta$-hydroxyvaleric acid and so forth; coryneform bacteria exhibiting auxotrophy for L-histidine, L-proline, L-threonine, L-isoleucine, L-methionine or L-tryptophan in addition to the resistance to 2-thiazolealanine (Japanese Patent Laid-open No. 54-44096); coryneform bacteria resistant to ketomalonic acid, fluoromalonic acid or monofluoroacetic acid (Japanese Patent Laid-open No. 57-18989); coryneform bacteria resistant to argininol (Japanese Patent Laid-open No. 62-24075); coryneform bacteria resistant to X-guanidine (X represents a derivative of fatty acid or aliphatic chain, Japanese Patent Laid-open No. 2-186995) and so forth.

Specifically, there can be mentioned *Brevibacterium flavum* AJ11169 (FERM BP-6892), *Corynebacterium glutamicum* AJ12092 (FERM BP-6906), *Brevibacterium flavum* AJ11336 (FERM BP-6893), *Brevibacterium flavum* AJ11345 (FERM BP-6894) and *Brevibacterium lactofermentum* AJ12430 (FERM BP-2228). The AJ11169 strain and the AJ12092 strain are the 2-thiazolealanine resistant strains mentioned in Japanese Patent Laid-open No. 54-44096, the AJ11336 strain is the strain having argininol resistance and sulfadiazine resistance mentioned in Japanese Patent Publication No. 62-24075, the AJ11345 strain is the strain having argininol resistance, 2-thiazolealanine resistance, sulfaguanidine resistance, and exhibiting histidine auxotrophy mentioned in Japanese Patent Publication No. 62-24075, and the AJ12430 strain is the strain having octylguanidine resistance and 2-thiazolealanine resistance mentioned in Japanese Patent Laid-open No. 2-186995.

Examples of *Escherichia* bacteria having L-arginine producing ability include *Escherichia coli* introduced with the argA gene (see Japanese Patent Laid-open No. 57-5693), and examples of bacteria belonging to the genus *Serratia* having L-arginine producing ability include *Serratia marcescens* deficient in ability to metabolize arginine and exhibiting resistance to arginine antagonists and canavanine and auxotorophy for lysine (see Japanese Patent Laid-open No. 52-8729).

Examples of coryneform bacteria having L-histidine producing ability include microorganisms belonging to the genus *Brevibacterium* and having resistance to a thiamin antagonist, specifically, *Brevibacterium lactofermentum* FERM P-2170, FERM P-2316, FERM P-6478, FERM P-6479, FERM P-6480 and FERM P-6481 (Japanese Patent Laid-open Publication No. 59-63194). Further, there can also be mentioned mutant strains belonging to the genus *Brevibacterium* or *Corynebacterium* and having resistance to polyketides and L-histidine producing ability, specifically, FERM P-4161, FERM P-7273, FERM P-8371, FERM P-8372 and ATCC14067.

Examples of bacterium belonging to the genus *Escherichia* having L-histidine producing ability include mutant strains belonging to the genus *Escherichia* and having resistance to a histidine analogue, for example, *Escherichia coli* R-344 strain, and bacteria belonging to the genus *Escherichia* introduced with an L-histidine synthesis system enzyme gene extracted from the foregoing strain. Specifically, there can be mentioned *Escherichia coli* NRRL-12116, NRRL-12118, NRRL-12119, NRRL-12120 and NRRL-12121 (Japanese Patent Laid-open Publication No. 56-5099).

Examples of bacteria belonging to the genus *Bacillus* having L-histidine producing ability include mutant strains belonging to the genus *Bacillus* and having resistance to a histidine analogue, and bacteria belonging to the genus *Bacillus* introduced with a gene obtained from the foregoing mutant strain and involved in resistance to histidine antagonist. Specifically, there can be mentioned FERM BP-218, FERM BP-224 and FERM BP-219 (Japanese Patent Laid-open Publication No. 58-107192).

In the present invention, a microorganism having an ability to produce a basic amino acid can be cultured in a liquid medium under an aerobic condition, for example and specifically, in the manner described below, in order utilize either or both of carbonate ions and hydrogencarbonate ions as main counter ions of the basic amino acid.

pH of the medium is controlled to be 6.5-9.0, preferably 6.5-8.0, during the culture, and 7.2-9.0 at the end of the culture. Further, a culture period where 2 g/L or more of hydrogencarbonate ions and/or carbonate ions exist in the medium is secured during the culture by controlling pressure in a fermentation tank to be a positive pressure during the fermentation, or supplying carbon dioxide gas or a mixed gas containing carbon dioxide gas to the medium. The expression of "a culture period where 2 g/L or more of hydrogencarbonate ions and/or carbonate ions exist in the medium is secured during the culture" used herein does not necessary means that the ions must exist in an amount of 2 g/L or more for the whole period of the culture, but means that it is sufficient that the ions exist in an amount of 2 g/L or more for any partial period of the culture. Preferably, the period where the ions exist in an amount of 2 g/L or more is a period of from logarithmic phase to stationary phase.

In the present invention, increase of the controlled pH shifts the equilibrium from one where the monovalent anion $HCO_3^-$ is dominant to one where the divalent anion $CO_3^{2-}$, which is more effective as the counter ion, is dominant. Furthermore, when pH is controlled with ammonia, increase of pH supplies ammonia, and it may become a source of nitrogen of the basic amino acid. As cations other than the basic amino acid, K, Na, Mg, Ca and so forth derived from the medium components can be mentioned. These cations account for 50% or less of the total cations.

In order to obtain a positive pressure in a fermentation tank, for example, a feed gas pressure higher than exhaust gas pressure can be used. By using a positive pressure in a fermentation tank, carbon dioxide gas produced by fermentation is dissolved in the culture broth to form hydrogencarbonate ions or carbonate ions, and these may serve as the counter ions of the basic amino acid. Specifically, the pressure in a fermentation tank may be 0.03-0.2 Mpa, preferably 0.05-0.15 MPa. Further, carbon dioxide gas may be dissolved in the culture broth by supplying carbon dioxide gas or a mixed gas containing carbon dioxide gas into the culture broth. Furthermore, the pressure in a fermentation tank may be controlled to become positive, while supplying carbon dioxide gas or a mixed gas containing carbon dioxide gas into the culture broth.

In order to obtain a positive pressure in a fermentation tank, for example, a feed gas pressure higher than exhaust gas pressure can be used. When carbon dioxide gas is supplied to the culture broth, the culture broth can be bubbled with pure carbon dioxide gas or a mixed gas containing 5 volume % or more of carbon dioxide gas.

The liquid medium used for the culture is not particularly limited, and any conventional known media containing organic or inorganic nutrient sources such as carbon source and nitrogen source and other trace amount nutrients can be used depending on a microorganism to be used. Any carbon source can be used so long as a microorganism can utilize it. For example, there can be mentioned sugars such as saccharose, glucose, fructose, molasses and starch hydrolysate, organic acids such as acetic acid, alcohols such as ethanol and so forth. As the nitrogen source, there can be mentioned inorganic substances such as ammonium ions, protein hydrolysate, yeast extract and so forth. As the trace amount nutrients, there can be mentioned amino acids, vitamins, trace amount metal elements and so forth.

Fermentation scheme is not also particularly limited, and it may be performed as any of batch culture where medium is not newly fed, feeding culture where medium is fed when initially added sugar is consumed, and continuous culture where medium is extracted when volume of the medium exceeds a volume acceptable for a fermentation tank.

While culture temperature may be suitably selected depending on the microorganism to be used, it is usually 25-45°C, preferably 30-40°C. Further, sufficient stirring is performed and sufficient oxygen is supplied during the fermentation.

In conventional methods, a sufficient amount of ammonium sulfate, ammonium chloride or protein decomposition product obtained with sulfuric acid or hydrochloric acid is added to the medium in order to use them as a source of counter anions for the produced basic amino acid, and thus the medium contains sulfate ions and chloride ions derived from those materials. Therefore, the concentration of carbonate ions, which shows weak acidity, is extremely low during the culture, and they exist at a ppm level. The present invention is characterized by reducing these sulfate ions and chloride ions, and dissolving carbon dioxide gas discharged from microorganisms during fermentation in the medium in the fermentation environment to use it as a source of the counter ions. Therefore, according to the present invention, it is not necessary to add sulfate ions or chloride ions exceeding their amounts necessary for the growth of the microorganism. Preferably, a suitable amount of ammonium sulfate or the like is fed to the medium in an early stage of the culture, and the feeding is stopped during the culture. Alternatively, ammonium sulfate or the like may be fed to the medium, while maintaining its good balance with respect to carbonate ions or hydrogencarbonate ions in the medium. Further, ammonia may be fed into the medium as a nitrogen source of L-lysine.

Usually, if ammonium sulfate is added to a medium as a counter ion source of basic amino acid, carbon dioxide in the culture broth will be discharged by sulfate ions. According to the present invention, in contrast, since it is not necessary to add an excessive amount of ammonium sulfate to the medium, carbon dioxide can be easily dissolved in the fermentation broth.

The fermentation broth, or a concentrate thereof, containing a basic amino acid obtained by the present invention contains carbonate ions or hydrogencarbonate ions at a concentration of 5 to 80% with respect to the normality of the basic amino acid produced by fermentation. These carbonate ions or hydrogencarbonate ions are discharged as carbon dioxide gas by heating. Therefore, the content of the basic amino acid in the solid components in the fermentation broth can be increased. Further, if an acid stronger than carbonic acid is added to the fermentation broth, it can easily substitute for the carbonic acid, and therefore various types of salts can be selected. The "fermentation product" includes a concentrate and dried product obtained form the aforementioned fermentation broth, the fermentation broth itself and dried product thereof.

The basic amino acid can be collected from the fermentation broth by a combination of known techniques such as ion exchange resin techniques, precipitation techniques and other techniques.

Examples

Hereafter, the present invention will be explained more specifically with reference to the following examples.

Example 1

(1) Seed culture of L-lysine producing bacterium

A medium containing 45 g/L of glucose, 15 g/L of molasses, 2 g/L (as nitrogen) of soybean protein hydrolysate, 2 g/L of $KH_2PO_4$, 5.6 g/L of NaOH, 10 g/L of ammonium sulfate, 0.8 g/L of $MgSO_4 \cdot 7H_2O$, 20 mg/L of $FeSO_4 \cdot 7H_2O$, 20 mg/L of $MnSO_4 \cdot 4H_2O$, 0.8 mg/L of thiamin hydrochloride and 0.2 mg/L of biotin (pH 6.0) was introduced into 1-L volume small glass fermentation tank in an amount of 300 mL, and sterilized by heating at 120°C for 20 minutes. After the fermentation the tank was cooled to 31.5°C, 5 platinum loops of *Brevibacterium lactofermentum* ATCC31269 preliminarily grown on an LB plate for 24 hours was inoculated to the medium, and cultured at 31.5°C and pH 7.0 for 30 hours with sufficient aeration and stirring.

(2) Main culture

A medium containing 30 g/L of glucose, 45 g/L of molasses, 2 g/L (as nitrogen) of soybean protein hydrolysate, 1.4 g/L of phosphoric acid, 1.2 g/L of NaOH, 30 g/L of ammonium sulfate, 1.5 g/L of $MgSO_4 \cdot 7H_2O$, 15 mg/L of $FeSO_4 \cdot 7H_2O$, 15 mg/L of $MnSO_4 \cdot 4H_2O$, 5 mg/L of thiamin hydrochloride and 0.75 mg/L of biotin (pH 5.0) was introduced into 1-L volume small glass fermentation tank in an amount of 300 mL, and sterilized by heating at 120°C for 20 minutes. After the fermentation the tank was cooled to 31.5°C, 45 mL of the above seed culture was inoculated to the medium, and cultured at 34°C with aeration of 1/2 vvm and sufficient stirring.

When the saccharide concentration in the culture broth became 5 g/L or less, a medium containing the following components was fed by the method described in Japanese Patent Laid-open Publication No. 5-30985. Specifically, pH and dissolved oxygen concentration were measured to detect depletion status of the carbon source based on changes of the measured values, and the medium was fed so as to maintain the concentration of the carbon source in the culture broth to be 5 g/L or less.

[Feed medium]

Medium containing 530 g/L of glucose, 1.4 g/L (as nitrogen) of soybean protein hydrolysate, 1.0 g/L of KOH, 44 g/L of ammonium chloride, 0.3 g/L of $MgSO_4 \cdot 7H_2O$, 0.35 mg/L of thiamin hydrochloride and 0.35 mg/L of biotin (pH 5.5)

After a predetermined amount of the feed medium was fed, the culture was finished when the saccharide in the culture broth was fully consumed. The culture was started at pH 7.0 and pH was gradually changed to 8.0. Simultaneously, the pressure in the tank was changed from 0 to 0.12 MPa.

As Comparative Example 1, ammonium sulfate was added instead of increasing pH and pressure in the tank.

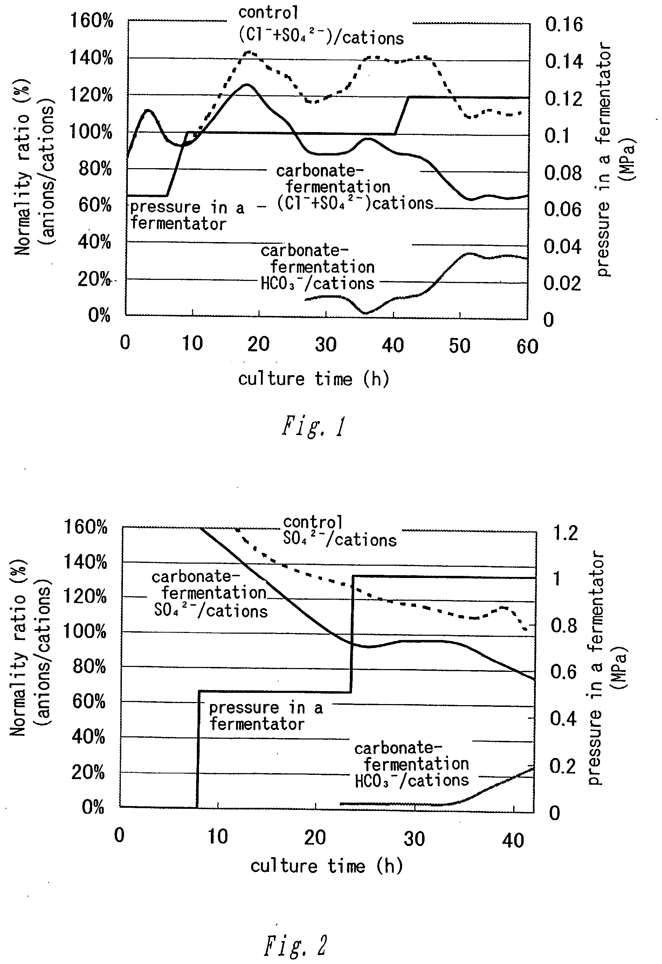

Major anion concentrations changed during the culture as shown in Fig. 1. While the sulfate ion concentration increased during the culture in Comparative Example 1, this concentration was low and the hydrogencarbonate ion concentration increased instead in Example 1.

After the completion of the culture, the normality ratio of carbonate ions and hydrogencarbonate ions was 33% with respect to cations mainly consisting of lysine, which was a basic amino acid in the fermentation broth. Further, the L-lysine content in the total dried product of the fermentation broth was 46%. On the other hand, in Comparative Example 1, the normality ratio of carbonate ions and hydrogencarbonate ions was 0% with respect to cations mainly consisting of lysine, and thus sulfate ions and chloride ions were excessive. Further, the L-lysine content in the total dried product of the fermentation broth was 43%.

Example 2

(1) Seed culture of L-lysine producing bacterium

A medium containing 40 g/L of glucose, 0.6 g/L (as nitrogen) of soybean protein hydrolysate, 1 g/L of $KH_2PO_4$, 5.6 g/L of NaOH, 8 g/L of ammonium sulfate, 1.0 g/L of $MgSO_4 \cdot 7H_2O$, 10 mg/L of $FeSO_4 \cdot 7H_2O$, 10 mg/L of $MnSO_4 \cdot 4H_2O$ (pH 6.0) was introduced into 1-L volume small glass fermentation tank in an amount of 300 mL, and sterilized by heating at 120°C for 20 minutes. After the fermentation the tank was cooled to 37°C, 5 platinum loops of *Escherichia coli* W3110(tyrA)/pCABD2 (WO95/16042) preliminarily grown on an LB plate for 24 hours was inoculated to the medium, and cultured at 37°C and pH 6.7 for 24 hours with sufficient aeration and stirring.

(2) Main culture

A medium containing 30 g/L of glucose, 0.4 g/L (as nitrogen) of soybean protein hydrolysate, 0.5 g/L of $KH_2PO_4$, 20 g/L of ammonium sulfate, 1.0 g/L of $MgSO_4 \cdot 7H_2O$, 30 mg/L of $FeSO_4 \cdot 7H_2O$ and 30 mg/L of $MnSO_4 \cdot 4H_2O$ (pH 5.0) was introduced into 1-L volume small glass fermentation tank in an amount of 300 mL, and sterilized by heating at 120°C for 20 minutes. After the fermentation the tank was cooled to 37°C, 50 mL of the above seed culture was inoculated to the medium, and cultured at 37°C with aeration of 1/2 vvm and sufficient stirring.

When the saccharide concentration in the culture broth became 5 g/L or less, a solution containing 760 g/L of glucose was fed by the method described in Japanese Patent Laid-open Publication No. 5-30985 in the same manner as in Example 1.

After a predetermined amount of the feed medium was fed, the culture was finished when the saccharide in the culture broth was fully consumed. The culture was started at pH 6.7 and pH was gradually changed to 8.0. Simultaneously, the pressure in the tank was changed from 0 to 0.1 MPa.

As Comparative Example 2, ammonium sulfate was added instead of increasing pH and pressure in the tank.

Major anion concentrations changed during the culture as shown in Fig. 2. While the sulfate ion concentration increased during the culture in Comparative Example 2, this concentration was low and instead the hydrogencarbonate ion concentration increased in Example 2.

After the completion of the culture, the normality ratio of carbonate ions and hydrogencarbonate ions was 25% with respect to cations mainly consisting of lysine, which was a basic amino acid in the fermentation broth. Further, the L-lysine content in the total dried product of the fermentation broth was 64%.

On the other hand, in Comparative Example 2, the normality ratio of carbonate ions and hydrogencarbonate ions was 0% with respect to cations mainly consisting of lysine, and thus sulfate ions and chloride ions were excessive. Further, the L-lysine content in the total dried product of the fermentation broth was 61%.

**Claims**

1. A fermentation broth containing a basic amino acid obtainable by culturing a microorganism having an ability to produce the basic amino acid in a liquid medium under an aerobic condition, or a concentrate thereof, wherein the fermentation broth and the concentrate thereof contain hydrogencarbonate ions and/or carbonate ions as counterions of cations mainly consisting of the basic amino acid in a normality ratio of 5% to 80%, which ratio is calculated according to the following equation:

```
normality ratio = [normality of hydrogencarbonate ions
and/or carbonate ions] / [normality of cations mainly
consisting of basic amino acid] x 100.
```

2. The fermentation broth containing a basic amino acid or the concentrate thereof according to claim 1, wherein the basic amino acid consists of one or more kinds of amino acids selected from L-lysine, L-arginine and L-histidine.

3. The fermentation broth containing a basic amino acid or the concentrate thereof according to any one of claims 1 and 2, wherein the microorganism is coryneform bacterium or *Escherichia coli.*

4. The fermentation broth containing a basic amino acid or the concentrate thereof according to claim 3, wherein the coryneform bacterium is *Corynebacterium glutamicum.*

5. A method for producing the fermentation broth containing a basic amino acid according to any one of claims 1 to 4 by fermentation, comprising
   the step of culturing a microorganism having an ability to produce the basic amino acid in a liquid medium under an aerobic condition to produce and accumulate the basic amino acid in the medium, wherein:

   pH of the medium is controlled to be 6.5-9.0 during the culture, and 7.2-9.0 at the end of the culture, and
   a culture period where 2 g/L or more of hydrogencarbonate ions and/or carbonate ions exist in the medium is secured during the culture by controlling pressure in a fermentation tank to be a positive pressure during the fermentation, or supplying carbon dioxide gas or a mixed gas containing carbon dioxide gas to the medium, wherein the pressure in the fermentation tank is 0.03-0.2 MPa,

so that the hydrogencarbonate ions and/or carbonate ions should serve as counter ions of cations mainly consisting of the basic amino acid.

6. The method according to Claim 5, wherein the basic amino acid consists of one or more kinds of amino acids selected from L-lysine, L-arginine and L-histidine.

7. The method according to any one of claims 5 and 6, wherein the microorganism is coryneform bacterium or *Escherichia coli.*

8. The method according to claim 7, wherein the coryneform bacterium is *Corynebacterium glutamicum.*


**Patentansprüche**

1. Fermentationsbrühe, enthaltend eine basische Aminosäure, erhältlich durch Kultivieren eines Mikroorganismus mit einer Fähigkeit, die basische Aminosäure in einem flüssigen Medium unter einer aeroben Bedingung zu produzieren, oder ein Konzentrat davon, wobei die Fermentationsbrühe und das Konzentrat davon Hydrogencarbonationen und/oder Carbonationen als Gegenionen von Kationen, hauptsächlich bestehend aus der basischen Aminosäure in einem Normalitätsverhältnis von 5% bis 80%, enthalten, wobei das Verhältnis nach folgender Gleichung berechnet wird:

Normalitätsverhältnis = [Normalität von Hydrogencarbonationen und/oder Carbonationen] / [Normalität von Kationen, hauptsächlich bestehend aus basischer Aminosäure] x 100.

2. Fermentationsbrühe, enthaltend eine basische Aminosäure oder das Konzentrat davon nach Anspruch 1, wobei die basische Aminosäure aus einer oder mehreren Arten von Aminosäuren besteht, ausgewählt aus L-Lysin, L-Arginin und L-Histidin.

3. Fermentationsbrühe, enthaltend eine basische Aminosäure oder das Konzentrat davon nach einem der Ansprüche 1 und 2, wobei der Mikroorganismus coryneformes Bakterium oder *Escherichia coli* ist.

4. Fermentationsbrühe, enthaltend eine basische Aminosäure oder das Konzentrat davon nach Anspruch 3, wobei das coryneforme Bakterium *Corynebacterium glutamicum* ist.

5. Verfahren zum Produzieren der Fermentationsbrühe, enthaltend eine basische Aminosäure nach einem der Ansprüche 1 bis 4, durch Fermentation, umfassend den Schritt des Kultivierens eines Mikroorganismus mit einer Fähigkeit, die basische Aminosäure in einem flüssigen Medium unter einer aeroben Bedingung zu produzieren und die basische Aminosäure in dem Medium zu akkumulieren, wobei:

der pH-Wert des Mediums kontrolliert wird, um 6,5 - 9,0 während der Kultur und 7,2 - 9,0 am Ende der Kultur zu sein, und
durch Kontrolle von Druck in einem Fermentationstank auf einen Überdruck während der Fermentation oder durch Zuführen von Kohlenstoffdioxidgas oder Mischgas, das Kohlenstoffdioxidgas enthält, zu dem Medium eine Kulturperiode, in der 2 g/L oder mehr an Hydrogencarbonationen und/oder Carbonationen im Medium vorhanden sind, während der Kultur gesichert ist, wobei der Druck in dem Fermentationstank 0,03 - 0,2 MPa ist, so dass die Hydrogencarbonationen und/oder Carbonationen als Gegenionen von Kationen, die hauptsächlich aus der basischen Aminosäure bestehen, dienen sollen.

6. Verfahren nach Anspruch 5, wobei die basische Aminosäure aus einer oder mehreren Arten von Aminosäuren besteht, ausgewählt aus L-Lysin, L-Arginin und L-Histidin.

7. Verfahren nach einem der Ansprüche 5 und 6, wobei der Mikroorganismus coryneformes Bakterium oder *Escherichia coli* ist.

8. Verfahren nach Anspruch 7, wobei das coryneforme Bakterium *Corynebacterium glutamicum* ist.

**Revendications**

1.  Bouillon de fermentation contenant un acide aminé basique pouvant être obtenu par la mise en culture d'un microorganisme ayant une capacité pour produire l'acide aminé basique dans un milieu liquide dans des conditions aérobies, ou un concentré de celui-ci, dans lequel le bouillon de fermentation et le concentré de celui-ci contiennent des ions hydrogénocarbonate et/ou des ions carbonate en tant que contre-ions de cations principalement constitués de l'acide aminé basique dans un taux de normalité de 5% à 80%, ledit ratio étant calculé selon l'équation suivante :

    taux de normalité = [normalité d'ions hydrogénocarbonate et/ou d'ions carbonate] / [normalité de cations principalement constitués d'acide aminé basique] x 100.

2.  Bouillon de fermentation contenant un acide aminé basique ou le concentré de celui-ci selon la revendication 1, dans lequel l'acide aminé basique consiste en un ou plusieurs types d'acides aminés choisis parmi la L-lysine, l'arginine et la L-histidine.

3.  Bouillon de fermentation contenant un acide aminé basique ou le concentré de celui-ci selon l'une quelconque des revendications 1 et 2, dans lequel le microorganisme est une bactérie corynéforme ou *Escherichia coli.*

4.  Bouillon de fermentation contenant un acide aminé basique ou le concentré de celui-ci selon la revendication 3, dans lequel la bactérie corynéforme est *Corynebacterium glutamicum.*

5.  Procédé de production du bouillon de fermentation contenant un acide aminé basique selon l'une quelconque des revendications 1 à 4 par fermentation, comprenant :

    l'étape de mise en culture d'un microorganisme ayant une capacité pour produire l'acide aminé basique dans un milieu liquide dans des conditions aérobies pour fabriquer et accumuler l'acide aminé basique, dans lequel :

    le pH du milieu est contrôlé pour être de 6,5 à 9,0 pendant la culture, et de 7,2 à 9,0 à la fin de la culture, et une période de culture où 2 g/L ou plus d'ions hydrogénocarbonate et/ou d'ions carbonate existent dans le milieu est assurée pendant la culture en contrôlant la pression dans un réservoir de fermentation pour être une pression positive pendant la fermentation, ou en fournissant au milieu du dioxyde de carbone gazeux ou un gaz mixte contenant du dioxyde de carbone gazeux, dans lequel la pression dans le réservoir de fermentation est de 0,03 à 0,2 MPa,
    de sorte que les ions hydrogénocarbonate et/ou les ions carbonate devraient servir de contre-ions de cations principalement constitués de l'acide aminé basique.

6.  Procédé selon la revendication 5, dans lequel l'acide aminé basique consiste en un ou plusieurs types d'acides aminés choisis parmi la L-lysine, la L-arginine et la L-histidine.

7.  Procédé selon l'une des revendications 5 ou 6, dans lequel le microorganisme est une bactérie corynéforme ou *Escherichia coli.*

8.  Procédé selon la revendication 7, dans lequel la bactérie corynéforme est *Corynebacterium glutamicum.*

*Fig. 1*

*Fig. 2*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5030985 A
- JP 5244969 A
- JP 11243985 A
- JP 48028078 A
- JP 56006499 A
- US 3708395 A
- US 3825472 A
- JP 50053588 A
- JP 50031093 A
- JP 52102498 A
- JP 53009394 A
- JP 53086089 A
- JP 55009783 A
- JP 55009759 A
- JP 56032995 A
- JP 56039778 A
- JP 53043591 A
- JP 53001833 A
- JP 55009784 A
- JP 56008692 A
- JP 53086090 A
- US 333455 A
- JP 56018596 A
- US 4346170 A
- WO 9516042 A
- EP 4884241992 A
- WO 9641871 A
- JP 54044096 A
- JP 57018989 A
- JP 62024075 A
- JP 2186995 A
- JP 57005693 A
- JP 52008729 A
- JP 59063194 A
- JP 56005099 A
- JP 58107192 A

**Non-patent literature cited in the description**

- *Int. J. Syst. Bacteriol.,* 1981, vol. 41, 255